(19) Europäisches Patentamt
European Patent Office
Office européen des brevets

(11) Publication number: **0 168 119**
**A1**

# (12) EUROPEAN PATENT APPLICATION

(21) Application number: **85201264.0**

(22) Date of filing: **07.04.83**

(51) Int. Cl.⁴: **C 07 D 321/00**
**C 07 J 17/00**

(30) Priority: **07.04.82 NL 8201492**

(43) Date of publication of application:
**15.01.86 Bulletin 86/3**

(84) Designated Contracting States:
**AT BE CH DE FR GB LI LU NL SE**

(60) Publication number of the earlier application in accordance with Art. 76 EPC: **0 106 863**

(71) Applicant: **RIJKSUNIVERSITEIT TE GRONINGEN**
**P.O. Box 72**
**NL-9700 AB Groningen(NL)**

(72) Inventor: **Hummelen, Jan Cornelis**
**Nieuwe Boteringestraat 76**
**NL-9712 PP Groningen(NL)**

(72) Inventor: **Meijer, Egbert Willem**
**Karel de Stoutelaan 8**
**NL-5583 XD Waalre-Aalst(NL)**

(72) Inventor: **Wynbert, Hans**
**Huygensweg 4**
**NL-9752 PA Haren(NL)**

(74) Representative: **van der Beek, George Frans et al,**
**Nederlandsch Octrooibureau Johan de Wittlaan 15 P.O. Box 29720**
**NL-2502 LS 's-Gravenhage(NL)**

(54) **Chemiluminescent substituted epidoxy-polycycloalkyl polycycloalkanes and process for preparing these compounds.**

(57) Chemiluminescent substituted epidioxy-polycycloalkyl polycycloalkanes of the formula

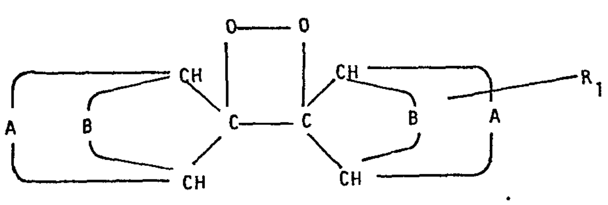

(1)

in which A and B represent alkylene radicals, which alkylene radicals may be attached to each other via an alkylene radical C, $R_1$ represents chloro, bromo, iodo, hydroxy, optionally substituted alkoxy, cycloalkoxy or acyloxy, or amino, acylamino, isothiocyanato, or isocyanato, with the proviso that in the case of a 4-eq.-$R_1$-2,2'-epidioxy-2,2'-adamantyl adamantane $R_1$ cannot be chloro or hydroxy.

These compounds are useful as thermochemiluminescent labels and probes in the study of biological processes and in immuno-assays.

Further, the invention relates to processes for preparing the compounds of formula 1.

EP 0 168 119 A1

Chemiluminescent substituted epidioxy-polycycloalkyl polycycloalkanes and process for preparing these compounds.

The invention relates to chemiluminescent substituted epidioxy-polycycloalkyl polycycloalkanes and to methods for preparing these compounds.

1,2-Epidioxy compounds, which are also called 1,2-dioxetanes are known to be thermochemiluminescent. The emitted chemiluminescence may be controlled by regulating the temperature of the system [T. Wilson in Int. Rev. Sci.: Phys. Chem. Ser. Two 9 (1976), 265]. 1,2-Epidioxy-adamantyl adamantanes are known to exhibit good stability, as appeared from a summary of a socalled "Poster Session" on bio- and chemiluminescence published in June 1981 by Academic Press, New York.

Further it is known to use luminescent labels and probes in analytical techniques used in biochemistry, clinical chemistry and biology for the qualitative and quantitative analysis and structural assay of biological materials. The chemiluminescent compounds, such as luminol and other phthalohydrazide derivatives used in these techniques have, however, the limitation that their chemiluminescence can be generated only by adding oxidants, for example hydrogen peroxide.

The present invention provides chemiluminescent compounds of formula 1

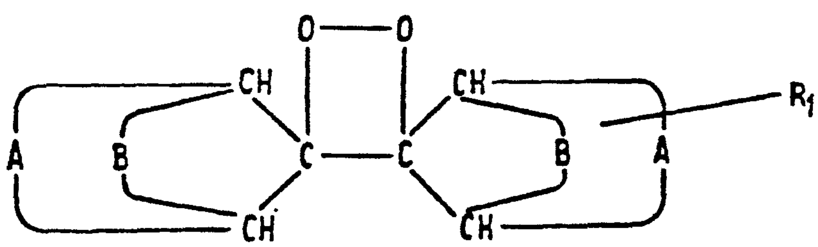

in which A and B represent alkylene radicals, which alkylene radicals may be attached to each other via an alkylene radical C and $R_1$ represents chloro, bromo or iodo, a hydroxy group, an optionally substituted alkoxy, cycloalkoxy or acyloxy group, or an amino, acylamino, isothiocyanato or isocyanato group, with the proviso that in the case of a 4-eq.-$R_1$-2,2'-epidioxy-2,2'-adamantyl adamantane $R_1$ cannot be chloro or hydroxy.

The compounds of formula 1 are useful as chemiluminescent labels

or can be regarded as labeled compounds, for example when the $R_1$ substituent is a radical of a biologically active compound, such as of a fatty acid, a steroid or a protein.

The novel chemiluminescent compounds according to the present invention are suitable for use in immunochemical methods of determination, in particular immunoassay, and in the study of steroids and membranes. Unlike known agents, such as luminol, the chemiluminescence is generated solely by heating.

In preferred compounds of formula 1, alkylene radical A contains 2 to 5 carbon atoms, alkylene radical B 2 to 5 carbon atoms, and alkylene radical C, if present, 1 to 4 carbon atoms. Examples of compounds of formula 1 are 4-eq.-$R_1$-2,2'-epidioxy-2,2'-adamantyl adamantanes, 4-eq.-$R_1$-9,9'-epidioxy-9,9'-bicyclo[3,3,1]-nonyl-bicyclo-[3,3,1]-nonanes, 2- or 7-$R_1$-8,8'-epidioxy-8,8'-bicyclo[3,2,1]-octyl-bicyclo[3,2,1]-octanes and 2- or 7-$R_1$-10,10'-epidioxy-10,10'-bicyclo[4,3,1]-decyl-bicyclo[4,3,1]-decanes, in which the two rings of each of the bicyclic radicals may be attached to each other via an alkylene bridge.

Preferably, the compounds of formula 1 are 4-eq.-$R_1$-substituted 2,2'-epidioxy-2,2'-adamantyl adamantanes.

Further, the invention relates to a process for preparing chemiluminescent compounds of formula 1, as defined above,

a. by subjecting a corresponding substituted polycycloalkylidene polycycloalkane of formula 2

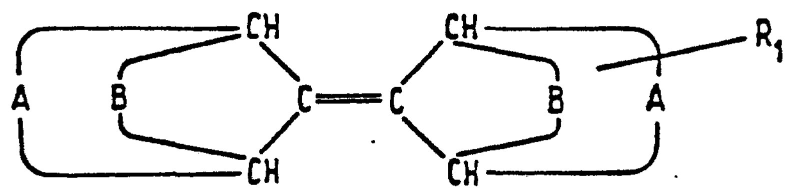

in which A and B represent alkylene radicals, which alkylene radicals may be attached to each other via an alkylene radical C, and $R_1$ represents chloro, bromo or iodo, a hydroxy group, an optionally substituted alkoxy, cycloalkoxy or acyloxy group, or an amino, acylamino, isothiocyanato or isocyanato group, with the proviso that in the case of a 4-eq.-$R_1$-2,2'-adamantylydene adamantane $R_1$ cannot be chloro or hydroxy,

to a photo-oxygenation reaction,

or

b. by converting, in a compound of formula 3,

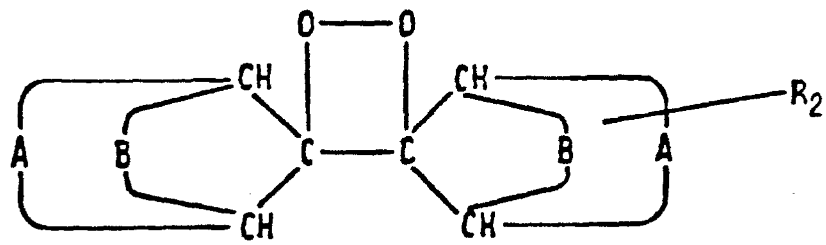

in which A and B represent alkylene radicals, which alkylene radicals may be attached to each other via an alkylene radical C, and $R_2$ represents chloro, bromo, or iodo, a hydroxy group, an optionally substituted alkoxy, cycloalkoxy or acyloxy group, or an amino, acylamino, thiocyanato or isocyanato group,

the substituent $R_2$ to a substituent $R_1$ within the meanings of $R_1$ given above.

Preferably, the photo-oxygenation reaction is carried out by irradiating a solution of 1-2 mmole of the starting material of formula 2 as defined above and 10-15 mg methylene blue in about 200 $cm^3$ $CH_2Cl_2$ with a sodium lamp and passing oxygen through the solution for 4-7 hours, decolourizing the solution with activated charcoal after termination of the reaction, and recovering the product after purification by column chromatography.

The thermochemiluminescent compounds according to the invention are particularly useful in membrane studies, in which they may be used as labels exhibiting chemiluminescence at a temperature of at least 150°C and up to approximately 250°C. By suitable substitution of the 1,2-dioxetane according to the invention, for example, by a long-chain fatty acid, the label is rendered compatible with the surroundings to be studied at the membrane.

A thermochemiluminescent fatty acid compound according to the invention is, for example, the compound of formula 4, in which the fatty acid radical is derived from arachidic acid. Such a compound has been found to have properties analogous to those of long-chain fatty acids, and to be suitable for use as a chemiluminescent label in membrane studies.

For chemiluminescence immunoassay based on a specific antibody-antigen reaction, a dioxetane compound according to the invention may be a "label", for example a protein label which is specific relative to certain functional groups in peptides.

Thus, for example, a protein may be marked at a free thiol group with a 4-eq.-substituted 2,2'-epidioxy-2,2'-adamantyl adamantane in which the substituent is α-iodoacetoxy or maleimido (formulae 5 and 6, respectively, of the sheet of formulae). The 1,2-dioxetane thus substituted may be reacted under standard conditions with, for example, Bovine Serum Albumine, which is a protein having 0.7 mole of free thiol groups per mole of protein. Purification of the reaction product by chromatography over a Sephadex G-10 column, dialysis against distilled water, and freeze drying produces the chemiluminescent protein. This procedure may also be applied to other proteins and, for example, to glutathione.

For immunoassay on the basis of chemiluminescence for steroid investigation, the 1,2-dioxetane compound according to the invention may contain, for example, a lithocholic acid (formula 7) or testosterone (formula 8) radical.

The invention is illustrated in and by the following examples. In the examples, melting points were determined by means of a Mettler FP2 melting point apparatus. IR spectra were recorded with a Unicam (SP-200) spectrophotometer and $^{1}H$ NMR spectra with a Varian A-60 of Hitachi Perkin Elmer R-24 B at 60 Mc. $^{1}H$ chemical shifts are given in $\delta$ units (ppm) relative to TMS (tetramethylsilane).

$^{13}CMR$ spectra were recorded at 25 Mc (Varian XL-100) and $^{13}C$ chemical shifts are indicated in $\delta$ units (ppm) relative to the solvent $CDCl_3$ and converted to $\delta$ TMS values using $\delta$ D $KCl_3$ = 76.9 ppm.

All solvents were purified and dried according to standard conditions.

The preparation of the starting materials of formula 2 is described in European Patent Application 83901235.8 (Publication number 0106863).

EXAMPLE I

Preparation of 4-eq.-bromo-2,2'-epidioxy-2,2'-adamantyl adamantane.

The bromo-substituted compound having formula 9 (1.5 mmol) was dissolved together with a pinch (approximately 12 mg) of methylene

blue in 200 $cm^3$ of distilled $CH_2Cl$. The reaction was carried out in a cylindrical glass reactor having at its bottom a porous P-4 glass filter and an inlet tube for oxygen such that oxygen can be introduced into the reactor via the filter. The top of the cylindrical reactor is provided with a supply vessel for the solvent (in this case $CH_2Cl_2$) and with a spherical cooler which, in turn, carries on its top a glass tube of approximately 1 m length.

Oxygen ($O_2$) was passed through the reaction vessel, and then the solution was transferred to the vessel. When the solution was added to the reaction vessel, the oxygen stream became visible from the rising in the solution of finely divided bubbles.

The solution present in the reaction vessel was irradiated with a sodium vapour lamp (Philips SON 160 W) provided with a reflector and placed close to the reaction vessel, for 6 hours, with the liquid level being replenished from time to time with $CH_2Cl_2$ from the supply vessel. During the reaction the temperature of the solution rose to approximately 40°C. The course of the reaction was followed by means of thin-layer chromatography (aluminium oxide/$CH_2Cl_2$ or n-hexane).

After completion of the reaction, the solution was poured out and decolorized with activated charcoal. The reaction product was purified by column chromatography to produce 4-eq.-bromo-2,2'-epidioxy-2,2'-adamantyl adamantane.

EXAMPLE II.

Preparation of SYN and ANTI 4-eq.-[12-oxy-dodecanoic acid methyl ester]-adamantylidene-adamantane-1,2-dioxetane (formula 11).

A solution of 240 mg of the compound having formula 10 and 15 mg methylene blue in 200 $cm^3$ $CH_2Cl_2$ was irradiated with a high-pressure mercury lamp, while a slow stream of oxygen was passed through the solution. The UV light was filtered with a $K_2Cr_2O_7$ solution. The reaction was followed by gas chromatographic analysis. A reaction period of 7 hours was required for complete conversion of the olefin to the 1,2-dioxetane having formula 11 . The dichloromethane was evaporated from the reaction mixture, which was then purified over an $Al_2O_3$ (II, III) column with $CH_2Cl_2$. The yield of compound having formula 11 in the form

of a colourless oil was 240 mg (94%): IR neat: 2900, 1715, 1460 and 1100 $cm^{-1}$; $^{1}H$ NMR ($CDCl_3$): $\delta$ 3.60 (S 3H); 3.6-3.2 (br.m., 3H); 2.8-2.4 (br. m., 4H); 2.4-1.0 (br. m., 42H); $^{13}C$ NMR ($CDCl_3$): $\delta$ 174.1 (s); 96.4 (s); 960 (s); 95.6 (s); 95.5 (s); 76.0 (d); 67.9 (d); 67.7; 51.2 (q) and 24 lines between 37.0 and 24.8 ppm.

EXAMPLE III.

Preparation of SYN and ANTI 4-eq.-[12-oxy-dodecanoic acid] adamantylidene-adamantane-1,2-dioxetane (formula 12).

To a solution of 240 mg of the compound having formula 11 in 15 $cm^3$ ethanol, a solution of 250 mg LiOH in 3 $cm^3$ water was added. The reaction mixture was stirred at room temperature for 12 hours. After acidification (pH = 4) with 0.1 N $H_2SO_4$, the reaction mixture was extracted with $CH_2Cl_2$ (2 x 30 $cm^3$). The organic layer was dried by means of $MgSO_4$, and concentrated. The yield of compound having formula 12 in the form of a colourless oil was 216 mg (92%): IR neat: 2900, 2500-3500, 1700, 1460, 1260, 1220 $cm^{-1}$; $^{1}H$ NMR ($CDCl_3$) $\delta$ 10.2-9.9 (s, 1H); 3.7-3.2 (br. m., 3H); 2.9-2.4 (br. m., 4H); 2.4-1.1 (br. m., 42H); $^{13}C$ NMR ($CDCl_3$): $\delta$ 179.7 (s); 95.8 (s); 95.4 (s); 95.3 (s); 75.9 (d); 75.7 (d); 67.8 (d); 67.5 (d) and 25 lines between 37.0 and 24.4 ppm.

EXAMPLE IV.

Preparation of 3-0-(4-eq.-2,2'-epidioxy-2,2'-adamantyl adamantane)-lithocholic acid methyl ester (formula 14 ).

A solution of 2.5 g of the compound having formula 13 and 50 mg methylene blue in approximately 350 $cm^3$ $CH_2Cl_2$ was irradiated with a high-pressure mercury lamp, while a slow stream of oxygen was passed through the solution. The UV light was filtered by means of a $K_2CR_2O_7$ solution. Complete conversion required a reaction period of 20 hours The solvent was evaporated from the reaction mixture, and the residue was purified over an $Al_2O_3$ (act. II/III) column with $CH_2Cl_2$.
The yield of compound having formula 14, in the form of white crystals, was 2.0 g (76 %). An analytically pure sample was obtained by crystallization from iso-propylalcohol; melting point: 119-121$^{o}$C; IR (nujol) 1740; 1160; 1100 $cm^{-1}$; $^{1}H$ NMR ($CDCl_3$) $\delta$ 3.7-3.4 (br.m., 1H); 3.54 (s, 3H); 3.4-3.0 (br.m. 1H); 2.7-2.3 (br.m. 4H); 2.3-0.4 (br.m. 53H); 0.77 (s, 3H); $^{13}C$ NMR ($CDCl_3$): $\delta$ 174.0 (s), 96.0 (s), 95.5 (s); 95.2 (s); 94.9 (s); 75.7 (d); 75.3 (d); 72.6 (d), 56.0 (d),

50.9 (q) and 28 lines between 42.3 and 11.6 ppm.; analysis $C_{45}H_{68}O_5$ calc.: 78.44%; 9.95% H; found 78.26% C; 9.99% H.

EXAMPLE V.

Preparation of 3-O-(4-eq.-2,2'-epidioxy-2,2'-adamantyl-adamantane)-lithocholic acid (formula 7).

To a solution of 200 mg of the compound having formula 14 in 25 $cm^3$ ethanol, 350 mg LiOH in 5 $cm^3$ water was added. The reaction mixture was stirred at room temperature for 12 hours. After acidification (pH = 4) with 0.1 N $H_2SO_4$, the reaction mixture was extracted with $CH_2Cl_2$. The organic layer was dried by means of $MgSO_4$ and concentrated. The yield of compound having formula 7 in the form of white crystals was 192 mg (98 %): melting point: 138-140°C; IR (Nujol): 3700-2700; 1705, 1090 $cm^{-1}$; $^1$H NMR ($CDCl_3$) $\delta$ 3.8-3.3 (br.m, 1H); 3.6-3.0 (br.m. 1H); 2.8-2.3 (br.m., 4H); 2.5-94 (br.m., 53H); 0.8 (s, 3H); 0.55 (s., 3H) and 10.1-10.7 (br.s., 1H); $^{13}$C NMR ($CDCl_3$): $\delta$ 180.3 (s); 96.4 (s); 59.9 (s); 95.7 (s); 95.5 (s); 75.7 (d); 75.4 (d); 72.9 (d); 56.2 (d); 55.8 (d) and 34 lines between 42.5 and 11.9 ppm.

EXAMPLE VI.

Preparation of 4-eq.-(maleimido)-2,2'-epidioxy-2,2'-adamantyl adamantane (2-isomers).

The olefin having formula 15 (500 mg) was subjected to photo-oxygenation in the manner specified before, except that the reaction period was 18 hours. Column chromatography over $Al_2O_3$ (act. III), using benzene as the eluent gave, after a first fast travelling impurity, the isomer having the formula 6a (75 mg) in the pure form and thereafter the isomer having formula 6b (60 mg) in the pure form, and both as white solids (together 25% yield).

Formula 6a:

$^1$H-NMR ($CDCl_3$, TSM): $\delta$ 6.5 (s, 2H), 4.2 (m. 1H), 3.4 (m, 1H), 2.9-2.5 (m, 4H), 2.4-1.0 (m, 21H). IR (KBr): 2950 (s), 1700 (s), 1365 (m), 1345 (m), 685 (m) $cm^{-1}$. $^{13}$C-NMR ($CDCl_3$): $\delta$ 171.7; 133.9; 95.8; 55.8; 37.1; 35.2; 34.4; 33.9; 33.0; 32.2; 31.9; 31.3; 30.9; 29.5; 27.2; 26.5; 26.3; 24.9.

Formula 6b:

$^1H$-NMR ($CDCl_3$, TMS): δ 6.55 (s, 2H), 4.15 (m, 1H), 3.4 (m, 1H), 2.9-2.4 (m, 4H), 2.4-1.0 (m, 21H). IR (KBr): 2900 (s), 1700 (s), 1375 (m), 690 (m) $cm^{-1}$. $^{13}C$-NMR ($CDCl_3$): δ 171.7; 133.9; 95.9; 55.3; 37.1; 34.5; 34.1; 33.7; 32.6; 31.9; 31.7; 31.5; 31.4; 31.1; 29.6; 28.8; 26.5; 26.3; 25.1.

EXAMPLE. VII.

Preparation of 4-eq.-(α-iodoacetoxy)-2,2'-epidioxy-2,2'-adamantyl adamantane (formula 5) ("anti-isomer").

200 mg of the compound having formula 16, 284 mg pro-analysis iodo acetic acid and 150 mg pro-analysis pyridine were together dissolved in 30 $cm^3$ distilled $CH_2Cl_2$. After the solution had been cooled to 0 °C, brought under a nitrogen atmosphere, and sealed from light, a solution of 330 mg DCC (dicyclohexyl carbodiimide) in 2 $cm^3$ $CH_2Cl_2$ was injected by means of a glass syringe with stirring. Without further cooling, the mixture was stirred for three days. After evaporation, 50 $cm^3$ benzene was added, the solution was filtered and the residue washed with benzene. The combined benzene solutions were washed with 5 % $Na_2S_2O_3$ solution (2 x 50 $cm^3$) and $H_2O$ (1 x 50 $cm^3$), dried by means of $MgSO_4$, filtered, and evaporated. The yellow solid residue was dissolved in pro-analysis acetone and the solution was partially decolorized by means of decolorizing charcoal. After filtration, the solution was thickened to about 25 $cm^3$ volume, subsequently a little water was added, the solution was filtered and then allowed to stand for 18 hours at -40 °C for crystallization. Filtration and drying produced 140 mg pure product (42 %); melting point 137.2-137.9 °C. $^1H$-NMR ($CDCl_3$, TMS): δ 5.15 (br, 1H); 3.75 (s, 2H): 3.1-2.4 (m, 4H); 2.4-1.1 (m, 22H). IR (KBr): 2900 (s), 1720 (s) and 1265 (s) $cm^{-1}$. $^{13}C$-NMR ($CDCl_3$): δ 167.9; 95.6; 95.2; 74.1; 36.9; 35.8; 34.7; 34.3; 32.9; 32.0; 31.8; 31.5; 31.4; 30.9; 30.5; 30.1; 26.5; 26.3; 25.3; -5.1.

The reaction to form 4-eq.-(α-iodoacetoxy)-2,2'-epidioxy-2,2'-adamantyl adamantane (formula 5) can also be carried out on a mixture of syn and anti hydroxydiadamantyl-1,2-dioxetane.

There is then formed a mixture of syn and anti α-iodoacetoxy compounds in the same yield. The I.R.- and the $^{1}H$-NMR-spectra are identical to those of the pure anti-isomer. $^{13}C$-NMR($CDCl_3$): δ 167.9; 167.5; 95.5; 95.7; 95.2; 74.3; 74.1 and 27 peaks between 39.1 and 25; -41.9 and -5.1.

The syn- and anti- 4-eq.-hydroxy-1,2-dioxetanes needed for the above synthesis, can be prepared from 4-eq.-hydroxyadamantylidene adamantane <u>via</u> the photo-oxygenation method in a quantitative yield (after purification by column chromatography over $Al_2O_3$ (act. II/III) using ether as the eluent. The separation of syn- and anti-isomers is effected by means of plate chromatography with $Al_2O_3$ and $CH_2Cl_2$ as the eluent.

CLAIMS.

1. Chemiluminescent compound of formula 1,

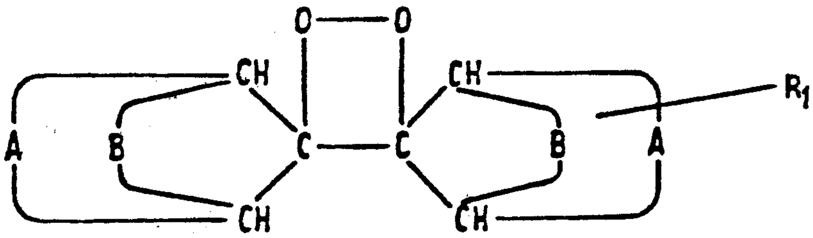

in which A and B represent alkylene radicals, which alkylene radicals may be attached to each other via an alkylene radical C and $R_1$ represents chloro, bromo or iodo, a hydroxy group, an optionally substituted alkoxy, cycloalkoxy or acyloxy group, or an amino, acylamino, isothiocyanato or isocyanato group, with the proviso that in the case of a 4-eq.-$R_1$-2,2'-epidioxy-2,2'-adamantyl adamantane $R_1$ cannot be chloro or hydroxy.

2. Compound according to claim 1 having formula 1, in which alkylene radical A contains 2 to 5 carbon atoms, alkylene radical B contains 2 to 5 carbon atoms, and alkylene radical C, if present, contains 1 to 4 carbon atoms.

3. 4-eq.-$R_1$-2,2'-epidioxy-2,2'-adamantyl adamantane in which $R_1$ represents bromo or iodo, an optionally substituted alkoxy, cycloalkoxy or acyloxy group, or an amino, acylamino, isothiocyanato or isocyanato group.

4. Compound according to any of claims 1-3 having formula 1, wherein $R_1$ represents an acyloxy group comprising a protein radical.

5. Compound according to claim 4, wherein the protein radical in $R_1$ is a radical of bovine serum albumine.

6. Compound according to any of claims 1-3 having formula 1, wherein a cycloalkoxy radical $R_1$ is a steroid radical attached via oxygen.

7. Compound according to claim 6, wherein the steroid radical is a testosterone or lithocholic acid radical.

8. Compound according to any of claims 1-3 having formula 1, wherein $R_1$ is a fatty acid radical attached via oxygen.

9. Compound according to claim 8, wherein the fatty acid radical is an arachidic acid radical.

10. A process for preparing chemiluminescent compounds, in which compounds of formula 1, as defined in claim 1 are prepared

a. by subjecting a corresponding substituted polycycloalkylidene polycycloalkane of formula 2

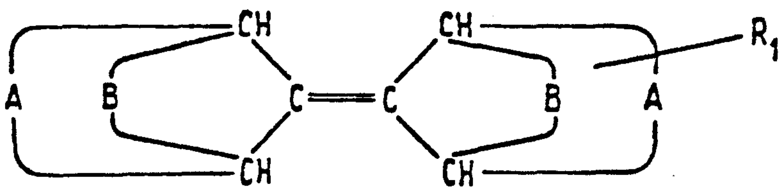

in which A and B represent alkylene radicals, which alkylene radicals may be attached to each other via an alkylene radical C, and $R_1$ represents chloro, bromo or iodo, a hydroxy group, an optionally substituted alkoxy, cycloalkoxy or acyloxy group, or an amino, acylamino, isothiocyanato or isocyanato group, with the proviso that in the case of a 4-eq.-$R_1$-2,2'-adamantylydene adamantane $R_1$ cannot be chloro or hydroxy,

to a photo-oxygenation reaction,

or

b. by converting, in a compound of formula 3,

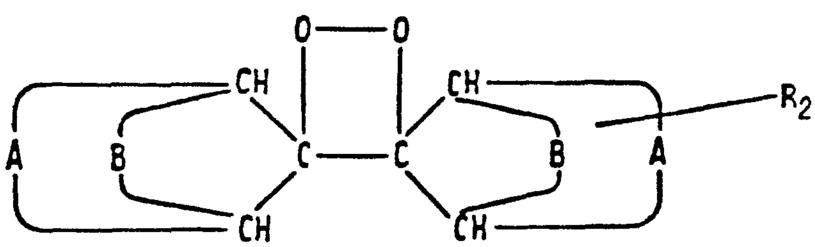

in which A and B represent alkylene radicals, which alkylene radicals may be attached to each other via an alkylene radical C, and $R_2$ represents chloro, bromo, or iodo, a hydroxy group, an optionally substituted alkoxy, cycloalkoxy or acyloxy group, or an amino, acylamino, thiocyanato or isocyanato group,

the substituent $R_2$ to a substituent $R_1$ within the meanings for $R_1$ given in claim 1.

11. The process of claim 10, in which the photo-oxygenation reaction is carried out by irradiating a solution of 1-2 mmole of the starting material of formula 2 as defined in claim 10 and 10-15 mg methylene blue in about 200 $cm^3$ $CH_2Cl_2$ with a sodium lamp and passing oxygen through the solution for 4-7 hours, decolourizing the solution with activated charcoal after termination of the reaction, and recovering the product after purification by column chromatography.

-----

0168119

1.

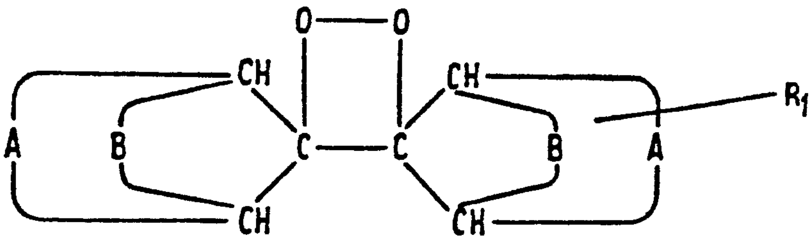

2.

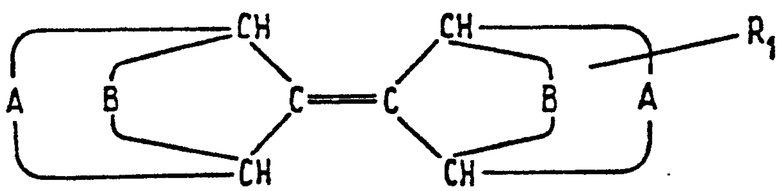

3.

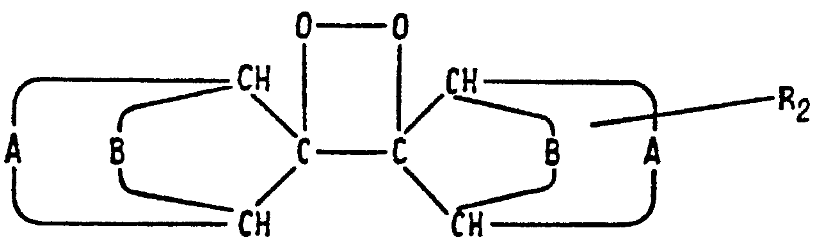

5.

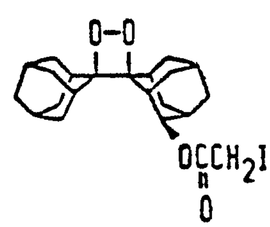

4.

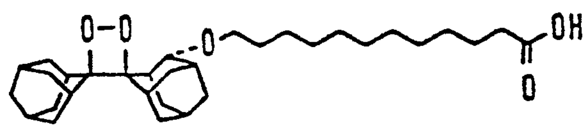

6a.

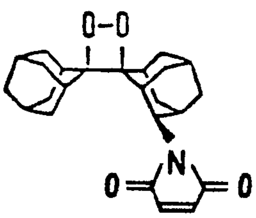

6b.

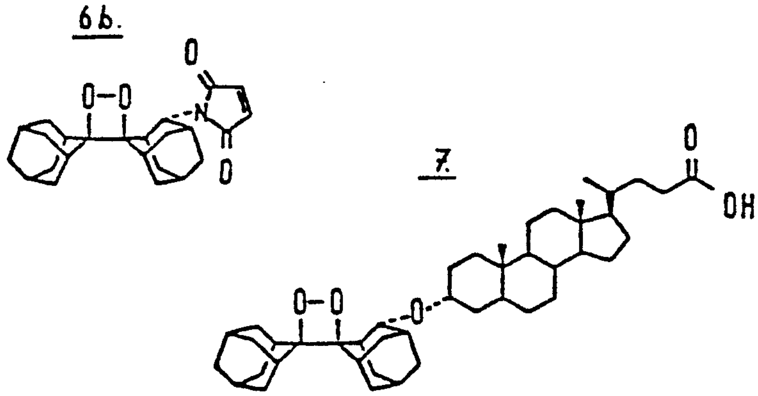

7.

8.

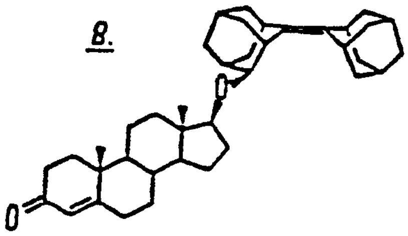

9.

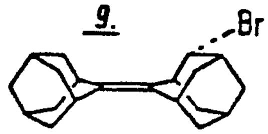

10.

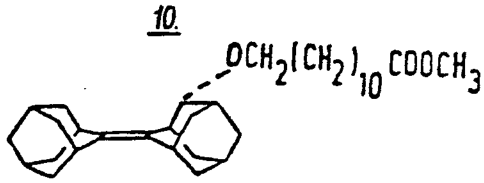

11.

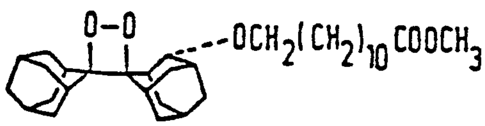

12.

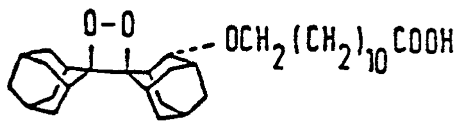

13.

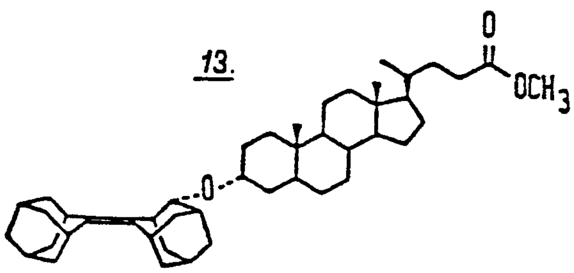

14.

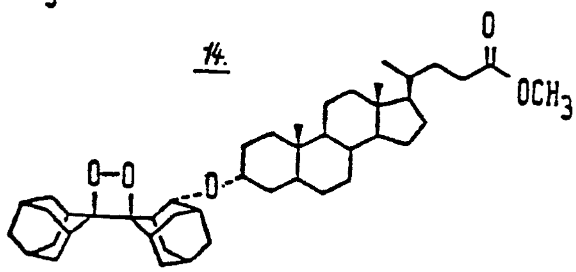

15.

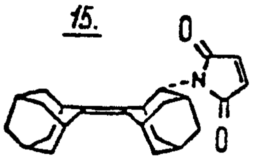

16.

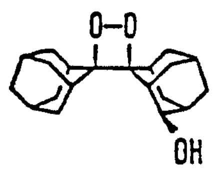

European Patent Office

# EUROPEAN SEARCH REPORT

0168119

Application number

EP 85 20 1264

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | TETRAHEDRON LETTERS, no. 2, 1972, pages 169-172, Pergamon Press, Oxford, GB; J.H. WIERINGA et al.: "Adamantylideneadamantane peroxide, a stable 1,2-dioxetane"<br>----- | 10,11 | C 07 D 321/00<br>C 07 J 17/00 |
| | | | TECHNICAL FIELDS SEARCHED (Int. Cl 4) |
| | | | C 07 D 321/00 |

The present search report has been drawn up for all claims

| Place of search | Date of completion of the search | Examiner |
|---|---|---|
| THE HAGUE | 24-09-1985 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82